# EUROPEAN PATENT APPLICATION

(11) **EP 3 175 920 A1**
(43) Date of publication of application: **07.06.2017**
(21) Application number: 15197780.8
(22) Date of filing: 03.12.2015
(51) Int. Cl.: B01L 3/00, A61K 38/17, A61K 35/16, A61K 38/20, C07K 14/54

(54) **METHOD AND DEVICES FOR PRODUCING SERUM**

(71) Applicant: Vetlinebio AB, 831 32 Östersund (SE)
(72) Inventor: LÖFSTRÖM, Lars, 2030 Nannestad (NO)
(74) Representative: Ström & Gulliksson AB

(57) **Abstract**

A method for obtaining a serum comprising IL-1Ra from a blood sample. In the method a blood sample is added to a sealed tube with a round-bottom and configured for receiving a blood sample. The tube further comprises a sealing cap provided with a valve and a sealing membrane. Furthermore the tube comprises a plurality of glass beads having a polished surface. After the blood sample has been added, the blood sample is agitated to induce the production of IL-1Ra. Thereafter, the serum comprising IL-1Ra is separated from the blood sample.

## Description

### Field of the Invention

This invention relates to serum with an improved ratio of anti-inflammatory to pro-inflammatory cytokines. More particularly this invention relates to a method of producing serum with improved ratio of growth factors and to a kit for use in producing an improved serum.

### Background of the invention

Orthopedic diseases are those that affect the body's musculoskeletal system. One such disease, osteoarthritis, relates to the chronic degeneration of joint structures e.g. cartilage, the synovial membrane, subchondral bone etc. It can affect humans and other animals such as horses and typically occurs in hands, knees, hips or spine. Osteoarthritis can also be known as degenerative arthritis or degenerative joint disease.

Orthopedic diseases and conditions such as osteoarthritis have traditionally been treated using one or a combination of surgery, non-steroidal anti-inflammatory drugs (NSAIDs), and analgesics. In the late 1970s and early 1980s the therapeutic use of cytokine inhibitors and growth factors was proposed. One important cytokine identified in orthopaedic diseases is Interleukin-1 (IL-1). Interleukin-1 receptor antagonist (IL-1Ra) is a competitive receptor antagonist of IL-1 with affinity to IL-1 receptors of both type I and II. IL-1Ra has an inhibitory effect on the pro-inflammatory effect of IL-1β and IL-1α (Perrier et al, FEBS Letters, 2006, 27:580). One hypothesis is that local IL-1Ra concentration is too low in degenerative diseases to inhibit the destruction of cartilage and other structures in the joint. The production and administration of serums and recombinant products/drugs comprising therapeutic levels of IL-1Ra have been proposed and used as a treatment for orthopedic diseases.

Methods of producing serums comprising therapeutic levels of cytokines such as IL-1Ra are known in the art. For instance, in US 6,713,246 blood from a mammal is incubated in a syringe to produce IL-1Ra.

The commercial products Orthokine® marketed by Orthogen AG is designed to induce IL-1Ra production in mammalian blood samples.

It was found in US 6,759,188 B2 that incubating blood in a syringe of which the surface has been treated with a corrosive agent to modify the surface could lead to improved production of IL-1Ra. It is thought that the increase in surface area promotes cytokine production.

EP 2 772 263 A1 discloses methods and devices for anabolic cytokine production. However, the devices require a special IL-1β binding coating to bind IL-1β and thus improve the ratio of IL-1Ra to IL-1β.

It would be ideal if a method of producing a serum from body fluids with further enhanced levels of Il-1Ra or an improved ratio of IL-1Ra to Il-1β was achievable. Furthermore, the efficiency of the production of serum from body fluids can be improved.

### Summary of the invention

Consequently, the present invention seeks to mitigate, alleviate, eliminate or circumvent one or more of the above-identified potential deficiencies in the art and disadvantages singly or in any combination by providing a method for obtaining serum comprising IL-1Ra from a blood sample. The method comprises adding a blood sample to a sealed tube configured for receiving a blood sample. The base of the tube has a round-bottom. The tube further comprises a sealing cap creating a substantially closed-system. The sealing cap has a valve and a sealing membrane. The tube further comprises a plurality of glass beads having a polished surface. After adding the sample to the tube the sample is then agitated to induce the production of IL-1Ra.

As opposed to the prevailing techniques the current method does not require complex surface modifications to the internal surface of the receptacle. The present method also provides significantly improved ratio of IL-1Ra to IL-1β.

### Brief Description of Drawings

Figure 1a is an isometric view of a tube according to an embodiment showing a cross-section of the sealing cap.
Figure 1b is a top-down isometric view of the tube showing the sealing cap.
Figure 2 illustrates cytokine concentration in serum for equine blood treated according to alternate methods as detailed in Experiment 2.
Figure 2a shows TNFα concentrations in pg/mL of serum obtained from a first batch of blood samples treated as detailed in Experiment 2.
Figure 2b shows IL-1β concentrations in pg/mL of serum obtained from a first batch of blood samples treated as detailed in Experiment 2.
Figure 2c shows IL-1Ra concentrations in pg/mL of serum obtained from a first batch of blood samples treated as detailed in Experiment 2.
Figure 2d shows concentration ratios of IL-1Ra to IL-1β in pg/mL:pg/mL of serum obtained from a first batch of blood samples treated as detailed in Experiment 2.
Figure 3 illustrates cytokine concentration in serum for equine blood treated as detailed in Experiment 3.
Figure 3a shows TNFα concentrations in pg/mL of serum obtained from a second batch of blood samples treated as detailed in Experiment 3.
Figure 3b shows IL-1β concentrations in pg/mL of serum obtained from a second batch of blood samples treated as detailed in Experiment 3.
Figure 3c shows IL-1Ra concentrations in pg/mL of serum obtained from a second batch of blood samples treated as detailed in Experiment 3.
Figure 3d shows concentration ratios of IL-1Ra to IL-1β in pg/mL:pg/mL of serum obtained from a second batch of blood samples treated as detailed in Experiment 3.
Figure 4 illustrates cytokine concentration in serum for equine blood treated with and without anti-coagulants.
Figure 5 illustrates the change in cytokine concentration within each group for the first batch of blood samples treated as detailed in Experiment 2.
Figure 5a shows the change in TNFα concentrations in pg/mL of serum obtained from a first batch of blood samples treated as detailed in Experiment 2.
Figure 5b shows the change in IL-1β concentrations in pg/mL of serum obtained from a first batch of blood samples treated as detailed in Experiment 2.
Figure 5c shows the change in IL-1Ra concentrations in pg/mL of serum obtained from a first batch of blood samples treated as detailed in Experiment 2.
Figure 5d shows the change in concentration ratios of IL-1Ra to IL-1β in pg/mL:pg/mL of serum obtained from a first batch of blood samples treated as detailed in Experiment 2.
Figure 6 illustrates the fold change in cytokine concentration within each group for the first batch of blood samples treated as detailed in Experiment 2.
Figure 6a shows the fold change in TNFα concentrations of serum obtained from a first batch of blood samples treated as detailed in Experiment 2.
Figure 6b shows the fold change in IL-1β concentrations of serum obtained from a first batch of blood samples treated as detailed in Experiment 2.
Figure 6c shows the fold change in IL-1Ra concentrations of serum obtained from a first batch of blood samples treated as detailed in Experiment 2.
Figure 6d shows the fold change in concentration ratios of IL-1Ra to IL-1β of serum obtained from a first batch of blood samples treated as detailed in Experiment 2.

### Detailed description of the invention

The following description of the present invention describes improved methods and devices for obtaining a serum comprising IL-1Ra from a blood sample. The inventor has identified that the physical and geometrical properties of the tube have a significant and hitherto unknown impact on the production of anti-inflammatory cytokines.

As detailed above previous techniques to increase the concentration of anti-inflammatory cytokines obtained in serum from blood samples have focused on increasing the surface area available to monocytes, the addition of pro-inflammatory binding components, or the addition of agents intended to increase the cytokine production.

In contrast to the above methods and techniques the inventors have developed an improved tube design. The improved tube design limits hemolysis, especially mechanical or shear stress induced hemolysis. A reduction in the mechanical stress experienced especially by monocytes during cytokine induction has been shown by the inventors to surprisingly result in an increased production of anti-inflammatory cytokines and an improved ratio of anti-inflammatory cytokines to pro-inflammatory cytokines. Many types of immune cells are reported to secrete IL-1ra including neutrophils, master cells, macrophages and monocytes (Hagaman DD, Okayama Y, D'Ambrosio C, Prussin C, Gilfillan AM, et al. (2001) Secretion of interleukin-1 receptor antagonist from human mast cells after immunoglobulin E-mediated activation and after segmental antigen challenge. Am J Respir Cell Mol Biol 25: 685-691; Malyak M, Smith MF Jr, Abel AA, Hance KR, Arend WP (1998) The differential production of three forms of IL-1 receptor antagonist by human neutrophils and monocytes. J Immunol 161: 2004-2010; Darragh J, Ananieva O, Courtney A, Elcombe S, Arthur JS (2010) MSK1 regulates the transcription of IL-1ra in response to TLR activation in macrophages. Biochem J 425: 595-602)

Not only is the absolute amount of anti-inflammatory of importance, but the ratio of anti-inflammatory cytokines to pro-inflammatory cytokines is also important, as an increased ratio of anti-inflammatory cytokines to pro-inflammatory cytokines has been shown to be beneficial (Hraha, T. H., Autologous conditioned serum: The comparative cytokine profiles of two commercial methods (IRAP and IRAP II) using equine blood, Equine Veterinary Journal 2011:43(5))

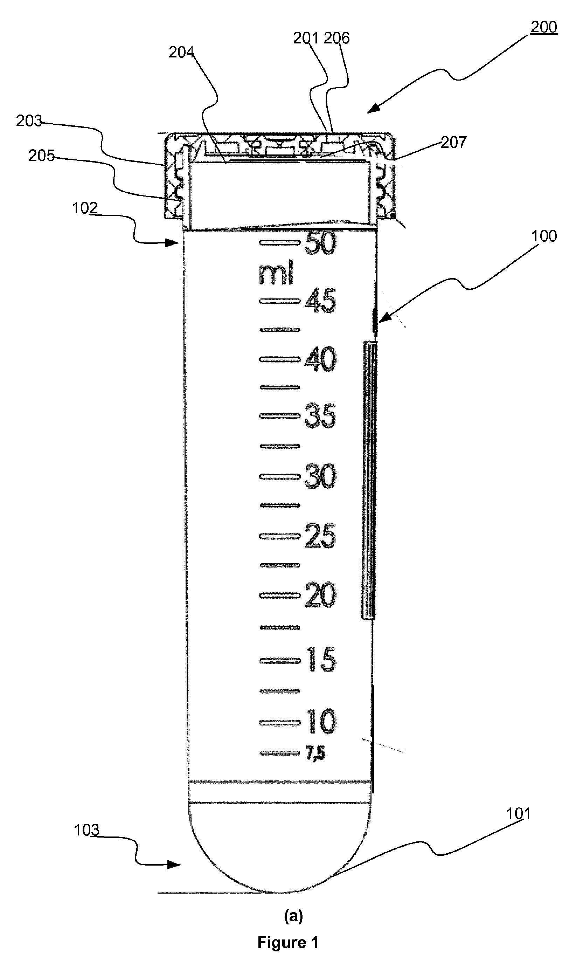

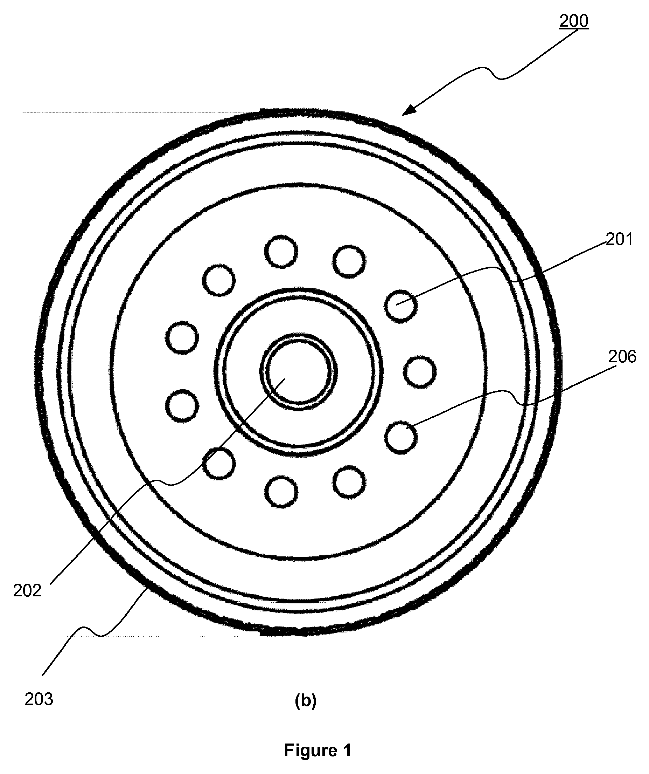

The inventors have developed an improved method of obtaining serum from a blood sample through the use of a sealed tube configured for receiving a blood sample. As can be seen in Figures 1a and b the base of the tube 100 is provided with a round-bottom 101. The round bottom 101 reduces hemolysis during filling and agitation. The round bottom tube may also protect against the lysis of especially monocytes in the blood during agitation.

The tube 100 is a cylindrical vessel having an opening at a 102 first end and a closed second end 103. As stated previously the closed end 103 of the tube is rounded 101, and may for example be formed by a spherical cap shape. In one embodiment the closed end is half-spherical. For clarification, the tube does not have a conical bottom or flat bottom.

The tube also comprises a sealing cap 200, the sealing cap 200 being provided with a valve 201 and a sealing membrane 202. The valve 201 can be formed by at least one opening 206 in the cap being sealed with a sterile hydrophobic filter 207 having a pore diameter of not more than 0.45µm, such as not more than 0.22 µm. Further, the pore diameter may be at least 0.05 µm. The valve functions such that air displaced during injection of the blood sample or aspiration of the serum passes through the valve, while preventing contamination of the sample. As can be seen in Figure 1b the sealing cap can be provided with a plurality of openings 206 through the cap 200. One end of the openings 206, at the inner surface of the cap 204, can be provided with a sterile hydrophobic filter. For example, a disc shaped filter having a central aperture. The central aperture can be positioned at the sealing membrane. As can be seen in Figure 1b the sealing membrane can be a septum. The septum can be manufactured from any suitable material such as silicone, polytetrafluroethylene (PTFE), polyethylene (PE), polypropylene (PP) or a combination of two or more of the above. In one embodiment the sealing membrane is pre-slit.

As can be seen in Figure 1a the sealing cap 200 can be a closure for a tube having an outer surface 203 and an inner surface 204. The inner surface 203 can be provided with threads 205 such that the cap can be screwed to a tube. The cap can be fixed to the tube in other ways such as, glued, moulded etc. At an annular region the outer surface may be provided with indentations and/or raised sections such that the cap can be better gripped by an operator's fingers.

The sealing cap 200 is provided at the open end of the tube 102. The tube being provided with a sealing cap results in a closed system allowing for more reliable handling of the tube and sample. The sealing cap furthermore negates the need for special handling processes such as a laminar flow hood.

The tube can be formed of a variety of suitable materials. For example, the tube can be a glass tube, such as a borosilicate glass. The tube may also be formed from a polymer. For example, the tube can be formed by polypropylene, polyethylene, polycarbonate or other transparent polymers.

The tube can further comprise a plurality of glass beads having a polished surface. The polished glass beads can have a diameter of approximately 3 mm, such as 3mm ± 1.5 mm. The glass beads can be borosilicate glass beads. The polishing of the glass beads can be performed in a wet drum system using a silicate polishing supplement. In one embodiment polished beads is considered to be beads having an optically smooth surface. In one embodiment polished beads is considered to be beads having an arithmetical mean surface roughness (Ra) of less than or equal to 50nm, such as less than or equal to 30nm. Further, the Ra may be at least 5nm, such as at least 10nm. The arithmetic mean surface roughness (Ra) can be obtained via measurement in accordance with International Standard, EN ISO 4287 and subsequent assessment/evaluation in accordance with EN ISO 4288.

A blood sample can be added to the tube configured for receiving a blood sample. The blood sample can be added such that hemolysis is avoided. In one embodiment, the blood sample can be added such that foaming of the sample is avoided, or at least kept at a minimum, such as by careful, slow injection towards the side wall of the tube. The blood sample can be added through the sealing membrane; typically by using a needle. The blood sample can be aspirated from a mammalian subject, such as a human, canine or equine subject.

After the blood sample has been added to the tube, the blood sample can be agitated to induce the production of IL-1Ra. In one embodiment the agitation can be performed by inverting the tube, or at least swaying it. For instance, the agitation could be performed by inverting the tube more than one time, such as more than 5 times, or such as. between 8 to 10 times. The agitation could be performed by a variety of different methods. Agitation of the blood sample should be performed gently. In one embodiment gently is considered to mean that any agitation of the tube according to the invention should not induce any substantial hemolysis. For example, the tube should not be shaken vigorously. After agitation the blood sample can be incubated. The tube can be incubated at 36 to 39 °C, such as 36.5 to 37.5 °C. In one embodiment the incubation is performed for not more than 24 hours. In one embodiment the incubation is performed for a period of 1 to 8 hours, such as approximately 4 hours. In some embodiments the incubation is performed by providing the tube to a pre-heated passive medium which prevents a fall in temperature (e.g., prevents a fall below 36 to 39 °C, such as 36.5 °C to 37.5 °C). For example, the tube can be provided to a pre-heated pad. In an embodiment passive medium is intended to describe mediums that can be pre-heated and then remain warm, i.e. within the desired temperature interval, for the duration of the incubation. The shorter incubation time allows a shorter total processing duration. With the present tube, even such a short incubation time has been found sufficient for substantial cytokine production. In some embodiments the blood sample does not need to be incubated. In such embodiments the plasma can be obtained directly after agitation and then allowed to clot to promote cytokine production. Due to the improved geometry of the tube a shorter duration of incubation is possible in a method according to the current invention. In one embodiment blood is provided to the tube such that it contacts the beads, shortly thereafter the blood is removed.

In one embodiment the tube is pre-heated prior to the provision of a blood sample. The tube can be pre-heated to a temperature of at least 35 °C. Pre-heating of the tube improves the ratio of anti-inflammatory to pro-inflammatory cytokines obtained. If the tube is not pre- heated it is possible that a fibrin clot occurs without a following platelet retraction resulting in a reduced amount of serum. If the blood sample is to be incubated the duration between the provision of the blood sample to the tube and the incubation should be as short as possible, such that a complete platelet caused clot retraction where serum is produced occurs. The beads may also be pre-heated within the tube. Any kit or components thereof may also be pre-heated as described herein. For example, syringes or tubing provided in a kit or other material necessary for the procedure may also be pre-heated.

The blood sample can be separated or fractionated in the tube to obtain a separate serum. In one embodiment the tube can be centrifuged at between 1200 and 3000 RCF (g), such as 2000 RCF (g).

In one embodiment the blood sample is fractionated in a first fractionation tube, such as a tube having a design according to the invention, but without glass beads. Subsequently the fraction comprising erythrocytes is discarded and the remaining fractions are transferred to a second induction tube, having a design according to the invention and comprising polished glass beads, for cytokine induction. In one embodiment fractionation occurs spontaneously in the fractionation tube. Whilst it may not be necessary the fractionation tube may be centrifuged to fractionate the blood sample, or to improve the fractionation result and achieve an improved serum.

As is stated above glass beads are not necessarily present in the fractionation tube.

In contrast to many of the existing methods an anti-coagulant is not provided to the blood sample. The internal surfaces of the tube are further generally not coated with an anti-coagulant. Anti-coagulants have even been shown to limit the IL-1Ra concentrations achievable as they prevent clotting which is necessary for production of a cytokine rich serum. In some embodiments at least one internal surface of the tube is coated with an anti-friction coating to further reduce mechanical stress on the components of the blood sample or serum thereby preventing triggering of the coagulation cascade. In one embodiment at least one of the internal surfaces of the tube, and/or the surface of the beads, are coated with at least one tethered-liquid-fluorocarbon (also denoted perfluorocarbon in the art), such as (Heptadecafluoro-1,1,2,2-tetrahydrododecyl)tris(dimetylamino)silane, Trifluoromethyltrimethylsilane, Nonafluorohexyltrimethoxysilane, (Heptadecafluoro-1,1,2,2-tetrahydrodecyl)trimethoxysilane, Trichloro(1H,1H,2H,2H-perfluorooctyl)silane and or perfluorodecalin. The sealing cap may also be treated with an anti-friction coating, such as that described above.

To remove the serum from the tube the tube can be aspirated through the sealing cap. In one embodiment the tube can be aspirated through the sealing cap with a needle connected to a syringe.

The tube is ideally sterile prior to receiving the blood sample. In one embodiment no further tissue or cellular material is added to the tube than the blood sample or erythrocyte reduced/depleted blood plasma. In an embodiment no biological material other than the blood sample is added to the tube. For example, the tube is free from added IL-1β binding antibodies, tissue or cellular material. Such addition of further tissue or cellular material increases the complexity of the method of obtaining serum.

In one embodiment the plurality of glass beads are treated with a liquid, such as an aqueous salt solution (e.g. an aqueous solution of NaCl), before adding the blood sample. For example a 1mL aqueous solution of NaCl can be provided to the tube prior to tube such that the beads contact the solution. The aqueous solution of sodium chloride can be sterile physiological saline e.g., a solution of sodium chloride 0.9% in sterile filtered double-distilled water. This has been shown to reduce any static charges which may be present on the glass beads.

In one embodiment an autologous serum obtained from the above process can be administered to a subject to treat an orthopaedic disease. The serum may be used in the treatment of inflammatory or non-inflammatory arthritis in a mammal, e.g. an equine subject. Further, the serum may be used in treatment of injuries of tendons and ligaments in a mammal, e.g. an equine subject.

For example, in one embodiment the blood sample is taken from an equine subject. The serum obtained from the method according to the invention can then be administered to the same equine subject. In some embodiments this serum is called autologous conditioned serum. In one embodiment the serum can be injected in to a joint of a horse to treat an orthopaedic disease, e.g. inflammatory or non-inflammatory arthritis.

Patients suffering from osteoarthritis and tendonitis may benefit from treatment with a serum obtained according to the invention. In veterinary applications, a blood sample from a horse may be used to produce prophylactically or therapeutically active serum.

Serum to be injected to a subject is filtered prior to injection. For example, the serum can be filtered with a 0.22µm filter.

The serum does not need to be autologous, it can alternatively be allogenic. In one embodiment serum obtained from a blood sample from a first donor is thereafter administered to a second donor. As is understood by a skilled person, in such a system immunological reactions would need to be suppressed or removed. An allogenic product has an advantage in that it may be less costly to obtain serum from non-autologous blood and may be provided as an off-the-shelf product i.e. being ready to use directly.

A serum obtained via a method according to the invention having an enhanced concentration of IL-1Ra can be used in the treatment of inflammatory or non-inflammatory arthritis in mammals e.g. an equine subject. Thus, an embodiment relates to a serum obtainable via the method according to the invention as well as the use of it in the treatment or prevention of inflammatory or non-inflammatory arthritis and/or the treatment of ligament and tendon injuries in a mammal. Similarly, another embodiment relates to a method of treating inflammatory or non-inflammatory arthritis and/or the treatment of ligament and/or tendon injuries in a mammal, the method including administration of a serum obtained via a method according to the invention. In such a method, a serum is administered, in a therapeutically effective dose, to a subject in need thereof. Further, another embodiment relates to the use of a serum obtained by a method according to the invention for the manufacture of a medicament, for use in the treatment of inflammatory or non-inflammatory arthritis and/or the treatment of ligament and tendon injuries in a mammal. The serum can be injected to the joint to be treated. The injected dosage will depend on a range of factors, for example, on the mass or type of the subject to be treated. In an embodiment a dose of approximately 4 mL can be injected to the fetlock joint of a horse. Serum obtainable via a method according to the invention has an especially high concentration ratio of IL-1Ra to IL-β. In one embodiment the serum can have a concentration ratio (mg/mL:mg/mL) of IL-1Ra to IL-β of greater than 350. In one embodiment the mean concentration ratio of IL-1Ra to IL-β of 10 serum samples is greater than 500, such as between 500 and 10,000, such as between 500 and 5,000.

Also provided in the current invention is a method for obtaining platelet-rich plasma (PRP) from a blood sample. A blood sample can be provided to a tube according to the invention, wherein the tube has a round bottom and is provided with a sealing cap having a valve and sealing membrane. The blood sample can be centrifuged in one or two steps to obtain platelet rich plasma of various compositions. It can then be incubated in the tube. The platelet-rich plasma can thereafter be further processed by initiating the coagulation cascade according to the above method to obtain a serum comprising IL-1Ra from a blood sample. However, the platelet-rich plasma is added to the tube instead of the blood sample.

In some embodiments the platelet-rich plasma can be administered directly to a subject, without further induction of IL-1Ra production to treat injuries and/or osteopathic diseases in a subject. For example, the platelet-rich plasma can be administered to equine subjects to treat various joint, ligament and tendon injuries.

Also provided according to the present invention, is a kit for obtaining a serum comprising IL-1Ra with a high ratio of anti-inflammatory cytokines to pro-inflammatory cytokines from a blood sample. The kit comprises a tube according to the invention, wherein the tube has a round bottom and is provided with a sealing cap having a valve and sealing membrane. Generally, the tube is pre-provided with glass beads. The kit further comprises a needle and syringe for extracting a venous blood sample. The kit also comprises a second needle and syringe for extraction of the serum from the tube and for administration into the subject. Ideally the administration syringe is provided with a 0.22µm filter at its tip prior to administration of the serum to the subject. While the kit may be sterilized by the user, the kit is typically provided in a sterile package ready to use. In one embodiment the kit is a kit for providing an equine-autologous conditioned serum.

Also provided according to the present invention is a tube comprising coated glass beads. The glass beads can be provided with particle coatings or layers to promote the production of therapeutically beneficial proteins in serum. Such beneficial proteins may include IL-1Ra and other anti-inflammatory cytokines such as IL-4, IL-6, IL-10, IL-11, and IL-13 and different growth factors such as transforming growth factor beta( TGF- β) and vascular endothelial growth factor (VEGF) . The beneficial proteins may be an actin-binding protein such as gelsolin. The beads may also be coated to hinder the production of pro-inflammatory proteins or cytokines.

The coating can be performed in addition to the polishing. In some embodiments the coated beads are however not polished. For example, in one embodiment the glass beads are be coated with silicon oxide particles. In one embodiment the glass beads are coated with gold particles having a size of between 0.01µm and 10µm. A coating of gold particles forming a layer applied to the glass beads may result in improved production of beneficial proteins, such as Gelsolin, in serum. It has been suggested that an increased concentration of Gelsolin in serum may be beneficial (DiNubile, *Plasma gelsolin as a biomarker of inflammation,* Arthritis Res Ther. 2008; 10(6): 124).

In further embodiments the glass beads are coated with noble metals such as Au, Ag, Pt, Rh, or Ir, or alloys thereof; with metals forming biocompatible oxides Zr, and Ti, or alloys thereof; with metals forming corrosion resistant oxides Al or Cr or alloys thereof; with W or Mo, or alloys thereof; or with bioactive materials such as Cu, Fe and Co. In some embodiments the glass beads are also coated with oxides such as TiO₂, ZrO₂, Cr₂O₃, Al₂O₃, SnO₂, In₂O₃ and MgO. The coatings can be in the form of particle coatings, thin films or layers.

The glass beads can also be coated with a combination of two or more of any of the above. In some embodiments the tube may comprise a mixture of polished glass beads having no particle coating, and beads having a particle coating. By including a mixture it may be possible to promote or hinder the production of specific cytokines.

Without further elaboration, it is believed that one skilled in the art can, using the preceding description and the following experimental part, utilize the present invention to its fullest extent. The preferred specific embodiments described herein are, therefore, to be construed as merely illustrative and not limitative of the remainder of the description in any way whatsoever. Further, although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

### Experimental data

The following examples are mere examples and should by no mean be interpreted to limit the scope of the invention. Rather, the invention is limited only by the accompanying claims.

### Experiment 1: Production of a serum comprising IL-1Ra from a blood sample.

### Preparation

A kit for obtaining a serum for IL-1Ra from a blood sample was provided. The kit comprised a tube according to the invention (cf. Fig. 1), a syringe (50 mL), a hypodermic sampling needle (18G: 1.2mm x 40 mm), a blunt injection needle (16G x 3.5") in an unopened and sterile package.

### Blood sample

A sample of 40 mL of venous blood was aspirated from a horse into a sterile syringe via a sterile 18G needle (1.2 mm x 40 mm).

### Addition of sample to tube

The sample of venous blood was then added to a tube according to the present invention. The tube comprised a round bottom as shown in Figure 1. The tube furthermore, comprised polished glass beads (Sigmund Lindner GmbH, SiLibeads Glass beads Type P Borosilicate 3mm). The blood sample was injected directly towards the internal wall of the tube to avoid foaming as much as possible.

### Agitation of the sample

The tube including the blood sample was then agitated by gentle inversion 8 times to induce the production of IL-1Ra.

### Incubation

The tube including the blood sample was then incubated for 24 hours at 37 °C.

### Separation

The blood sample was fractionated to obtain a separate serum. Centrifugation was performed at 2000 RCF (g). This relative centrifugal force is equivalent to 4000RPM in a Hettich Centrifuge or 3667RPM in a Hermle centrifuge. The centrifuge was balanced with a counter-weight.

### Extraction

The tube was aspirated using a blunt injection needle (16G x 3.5") in cooperation with a 30 mL syringe. The serum comprising IL-1Ra and other cytokines was removed from the tube, passed through a 0.22 micron filter, and transferred to a second tube for analysis of cytokine levels as disclosed in experiment 2.

### Experiment 2: Comparison of serum obtained in tube according to the invention and serum obtained from a range of other tubes and processes.

### Sample collection and grouping

Five blood samples were aspirated from ten equine subjects. The five samples were processed in different tubes and according to different processes depending on the type of tube, length of incubation step, or the presence/surface of glass beads.

***Vacuette; 1hr*** (Vacuette^{®}, Greiner Bio-One): cytokine induction performed in a vacuette tube. Prior to cytokine induction the sample was not incubated. This sample formed the baseline or control sample.

***Vacuette; 24hr*** (Vacuette^{®}, Greiner Bio-One): cytokine induction performed in a vacuette tube. Prior to cytokine induction the sample was incubated for 24 hours.

***Type 1 beads; 6 hours*:** cytokine induction performed in a tube according to the invention, however, the tube comprised glass beads which had been treated to increase the bead surface area. Prior to cytokine induction the sample was incubated for 6 hours.

***Type 1 beads, 24 hours*:** cytokine induction performed in a tube according to the invention, however, the tube comprised glass beads which had been treated to increase the bead surface area. Prior to cytokine induction the sample was incubated for 24 hours.

***Type II beads*:** blood sample treated according to the process described in Experiment 1, in a tube according to the invention being provided with polished glass beads.

***PET Tubes:*** A further blood sample was aspirated from four of the equine subjects and processed in round bottom tubes according to the invention, however the tubes did not comprise any glass beads. Samples were transported on dry ice and stored at -20 °C.

### Cytokine induction

For each of the samples cytokine induction was performed. Agitation was performed as is described in Experiment 1. However, as is clear form the groupings described above, the induction differed with respect to the type of tube, the length of incubation, presence of glass beads, glass bead coating.

The label codes of the samples were unknown to the investigators.

### Cytokine analyses

Samples were analysed at the Royal Veterinary College, University of London.

TNFα concentration was determined via an equine TNFα ELISA kit (R&D Systems, UK. Catalogue number: DY1814). The samples were diluted as necessary as recommended by *Optimization of a procedure to accurately detect equine TNFα in serum samples*. Lavoie-Lamoureux A, Maghni K, Lavoie JP. Vet Immunol Immunopathol. 2010 Nov 15;138(1-2):118-23. Where necessary sample dilutions were performed with Bio-Plex Pro Isotyping diluent as recommended. A standard curve was produced for each plate using the software on a Tecan Magellan Pro plate reader. The samples were analysed in duplicate on a Tecan Magellan Pro plate reader. The concentration of TNFα in the 54 samples was then determined.

IL-1β concentration was determined via an equine IL-1β ELISA kit (R&D Systems, UK. Catalogue number: DY3340). A standard curve was produced for each plate using the software on a Tecan Magellan Pro plate reader. The samples were analysed in duplicate on a Tecan Magellan Pro plate reader. The concentration of IL-1β in the 54 samples was then determined.

IL-1Ra concentration was determined via an equine IL-1Ra ELISA kit (R&D Systems, UK. Catalogue number: DY2466). Where necessary sample dilutions were performed with Bio-Plex Pro Isotyping diluent as recommended. A standard curve was produced for each plate using the software on a Tecan Magellan Pro plate reader. The samples were analysed in duplicate on a Tecan Magellan Pro plate reader. The concentration of IL-1Ra in the 54 samples was then determined.

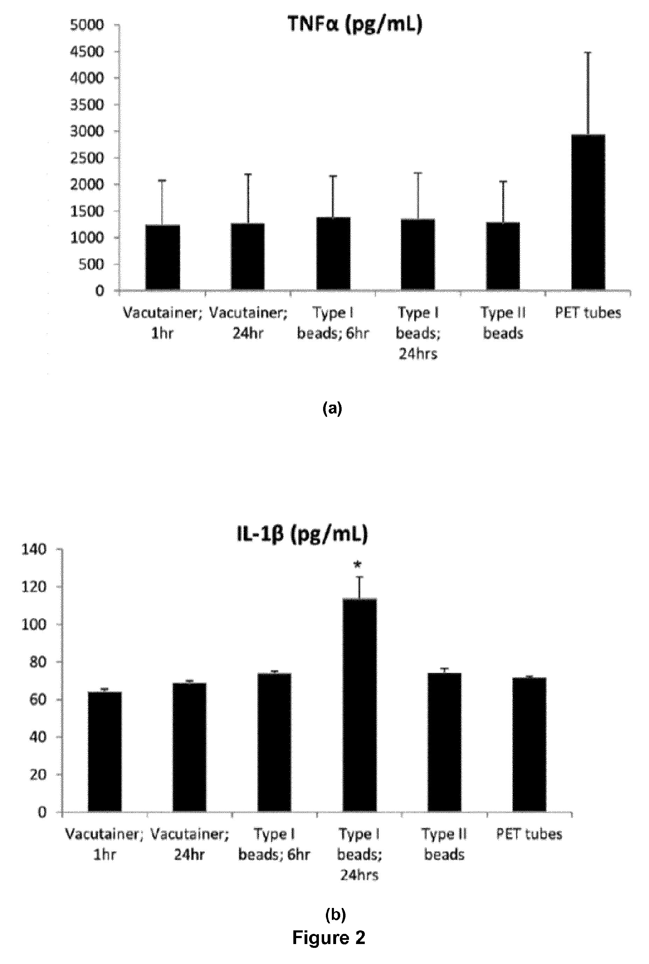

The Results of the analysis is provided in Figures 2a-d, Figures 5a-d and Figures 6a-d.

In Figures 2a-c results shown are concentration mean values ± SD for the 10 subjects of specific cytokines. The y-axis shows the concentration of the relevant cytokine in pg/mL, the x axis shows the different groups.

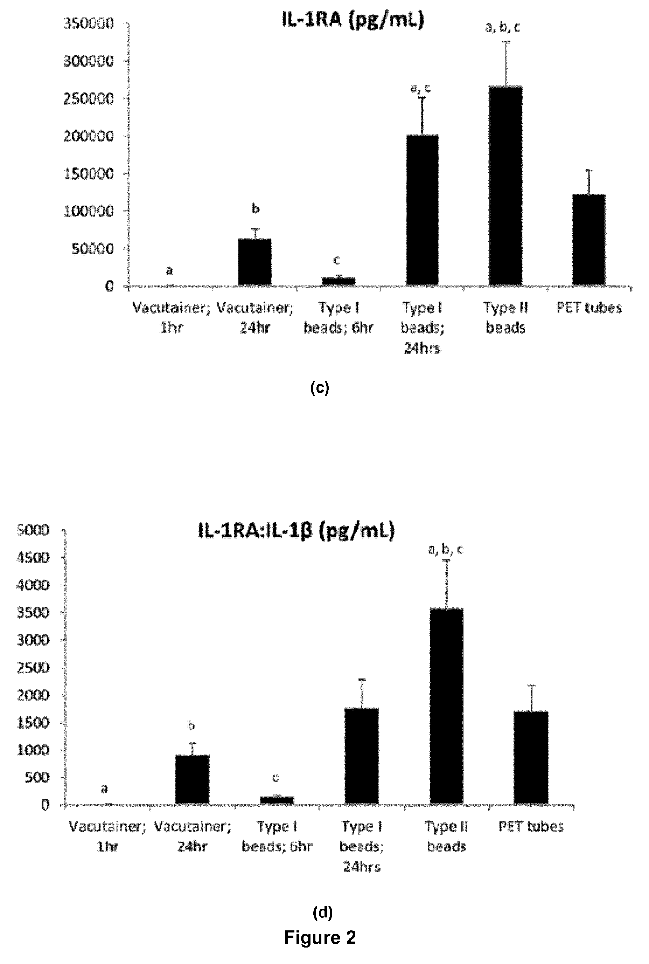

In Figure 2d the mean ratio ± SD of the concentration of IL-1Ra to IL-1β is shown. The y-axis shows the ratio in pg/mL:pg/mL, the x axis shows the different groups.

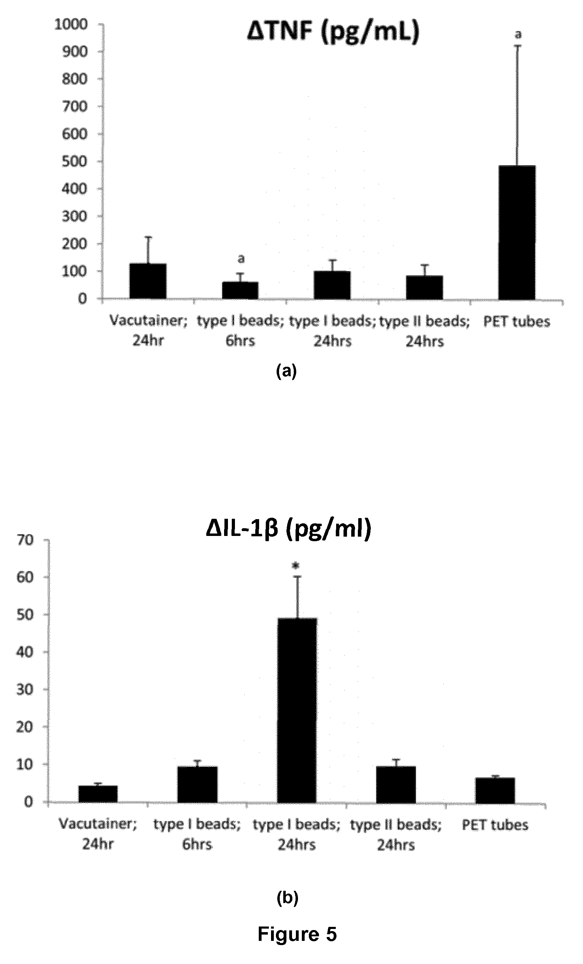

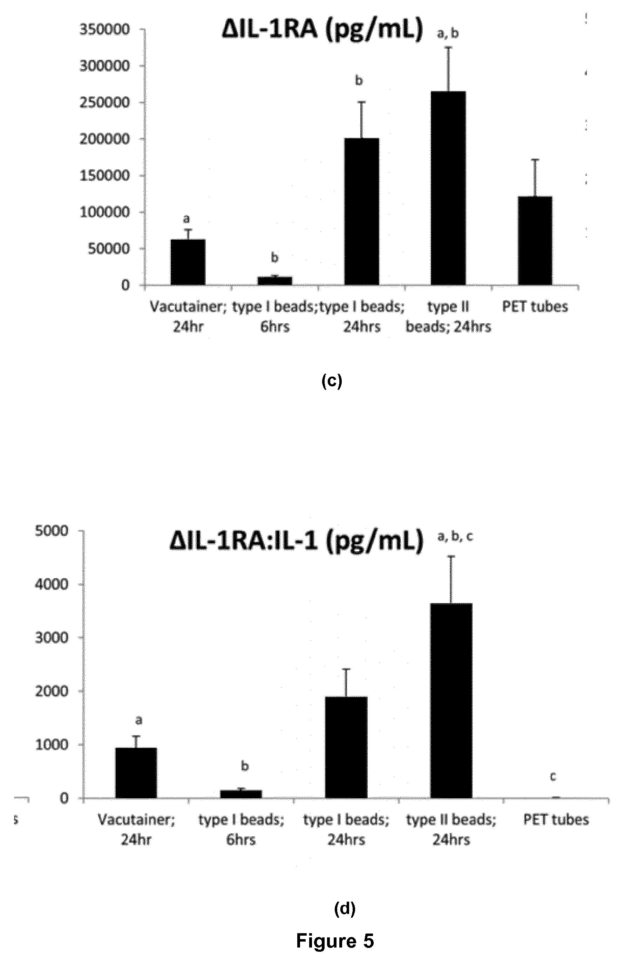

In Figure 5a-c results shown are the change in concentration mean values ± SD for the 10 subjects of specific cytokines. The y-axis shows the change in concentration of the relevant cytokine in pg/mL, the x axis shows the different groups.

In Figure 5d the mean ± SD of the change in concentration ratio of IL-1Ra to IL-1β is shown. The y-axis shows the ratio in pg/mL:pg/mL, the x axis shows the different groups.

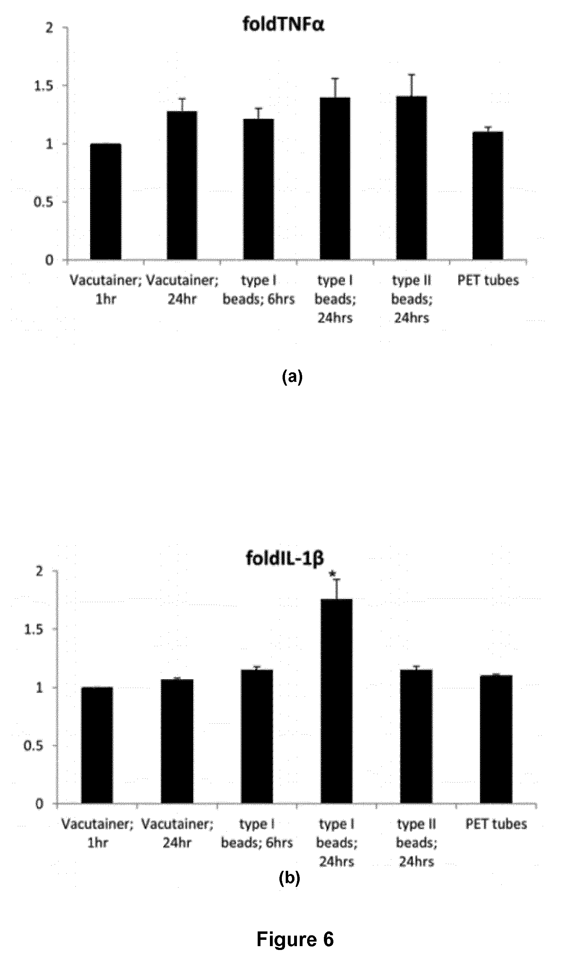

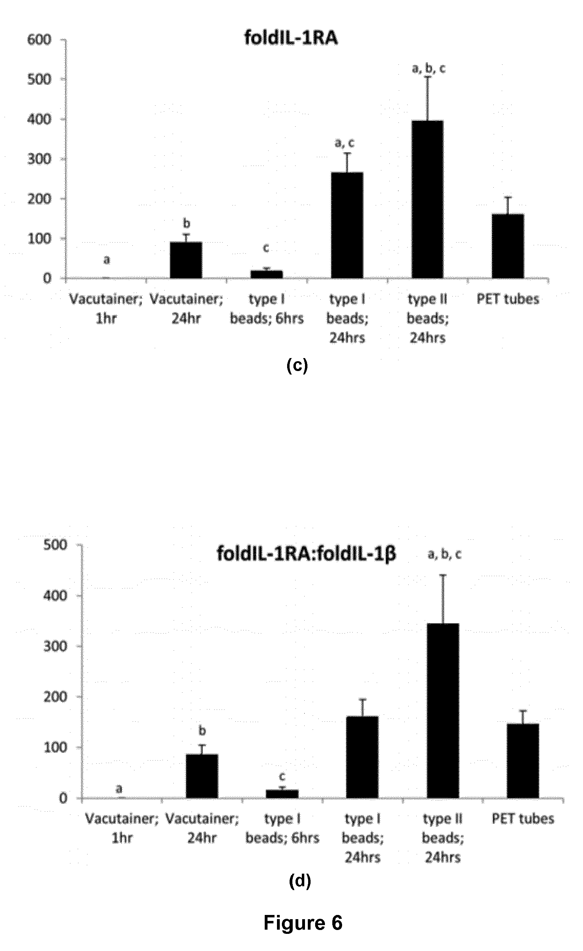

In Figure 6a-c results shown are the fold change in concentration mean values ± SD for the 10 subjects of specific cytokines. The y-axis shows the fold change in concentration of the relevant cytokine, the x axis shows the different groups.

In Figure 6d the mean ± SD of the fold change in concentration of IL-1Ra to IL-1β is shown. The y-axis shows the fold change of the ratio, the x axis shows the different groups.

### Experiment 3: Comparison of serum obtained in tube comprising polished beads but having a conical bottom and serum obtained via existing commercial solution.

### Sample collection, grouping and cytokine induction

Three blood samples of venous blood were aspirated from ten equine subjects. The three separate samples were obtained and treated according to the following:
a) IRAP, Instructions for use, Arthrex IRAP II, Arthrex Vet Systems, 2014
b) **Type II Beads,** the process described in Experiment 1, however, the tube differed from the tube according to the invention in that the had a conical bottom, and
c) **Type III Beads,** the process as described in Experiment 1, however, the glass beads were roughened such that their surface area was increased. The tubes had, furthermore, conical bottoms. The label codes of the samples were unknown to the investigators.

Samples were transported on dry ice and stored at -20 °C.

### Cytokine analyses

Samples were analysed at the Royal Veterinary College, University of London.

TNFα concentration was determined via an equine TNFα ELISA kit (R&D Systems, UK. Catalogue number: DY1814). The samples were diluted as necessary as recommended by *Optimization of a procedure to accurately detect equine TNFα in serum samples*. Lavoie-Lamoureux A, Maghni K, Lavoie JP. Vet Immunol Immunopathol. 2010 Nov 15;138(1-2):118-23. Where necessary sample dilutions were performed with Bio-Plex Pro Isotyping diluent as recommended. A standard curve was produced for each plate using the software on a Tecan Magellan Pro plate reader. The samples were analysed in duplicate on a Tecan Magellan Pro plate reader. The concentration of TNFα in the 30 samples was then determined.

IL-1β concentration was determined via an equine IL-1β ELISA kit (R&D Systems, UK. Catalogue number: DY3340). A standard curve was produced for each plate using the software on a Tecan Magellan Pro plate reader. The samples were analysed in duplicate on a Tecan Magellan Pro plate reader. The concentration of IL-1β in the 30 samples was then determined.

IL-1Ra concentration was determined via an equine IL-1Ra ELISA kit (R&D Systems, UK. Catalogue number: DY2466). Where necessary sample dilutions were performed with Bio-Plex Pro Isotyping diluent as recommended. A standard curve was produced for each plate using the software on a Tecan Magellan Pro plate reader. The samples were analysed in duplicate on a Tecan Magellan Pro plate reader. The concentration of IL-1Ra in the 30 samples was then determined.

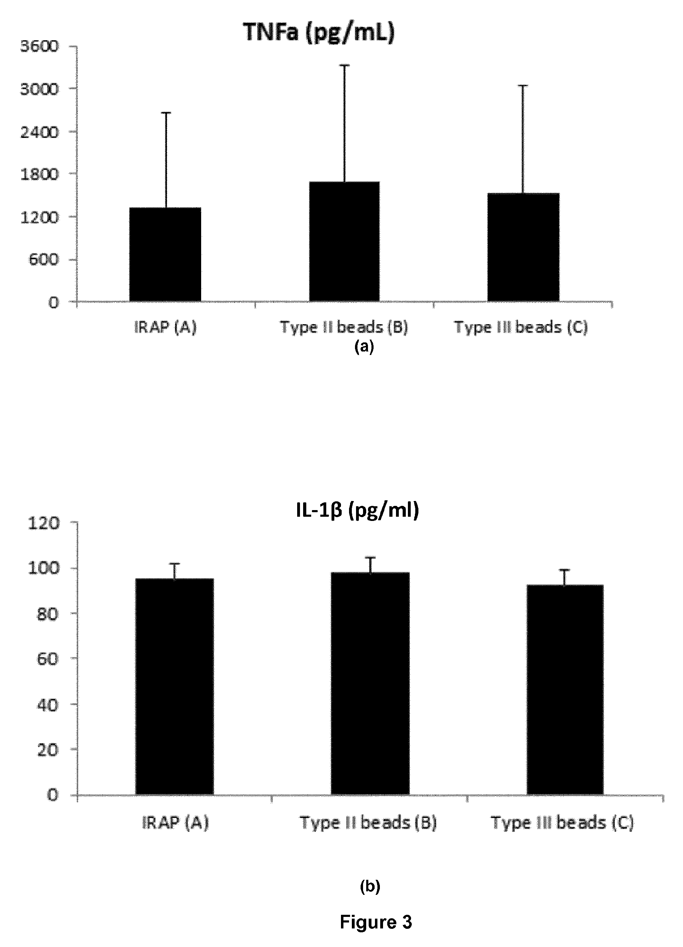

### The Results of the analysis is provided in Figures 3a-d.

In Figures 3a-c results shown are mean values ± SD for the 10 subjects. The y-axis shows the concentration of the relevant cytokine in pg/mL, the x axis shows the different groups.

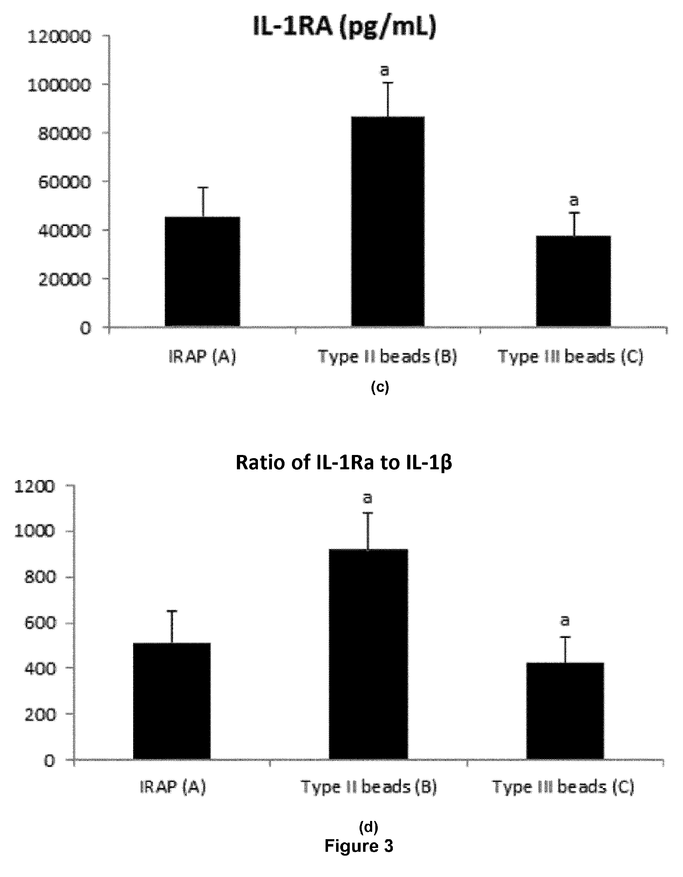

In Figure 3d the mean ratio ± SD of the concentration of IL-1Ra to IL-1β is shown. The y-axis shows the ratio in pg/mL:pg/mL, the x axis shows the different groups.

### Experiment 4: Serum obtained from blood treated with an anti-coagulant.

Four blood samples of venous blood were aspirated from three equine subjects. The three separate samples were obtained and treated according to the following:
***Type II Beads:*** the process described in Experiment 1, however, the tube differed from the tube according to the invention in that it had a conical bottom. The blood sample was incubated for 24 hours.
***Type II Beads* + *ACD-A*:** the process as described in Experiment 1, however, the tube differed from the tube according to the invention in that the tube had a conical bottom, the blood sample was furthermore treated with Anticoagulant Citrate Dextrose Solution, Solution A. The blood sample was incubated for 24 hours.
***Type II Beads*+*ACD*-*A* + *1 hr mixing:*** the process as described in Experiment 1, however, the tube differed from the tube according to the invention in that the tube had a conical bottom, the blood sample was furthermore mixed by alternately inverting and righting the tube for 1 hour. The blood sample was incubated for 23 hours.

***ACD-A only:*** the process as described in Experiment 1, however, the tube had a conical bottom and the tube did not comprise any glass beads. The blood sample was furthermore treated with 10% Anticoagulant Citrate Dextrose Solution, Solution A. The blood sample was incubated for 24 hours.

Cytokine analysis was performed in accordance with the procedure outlined in Experiments 2 and 3.

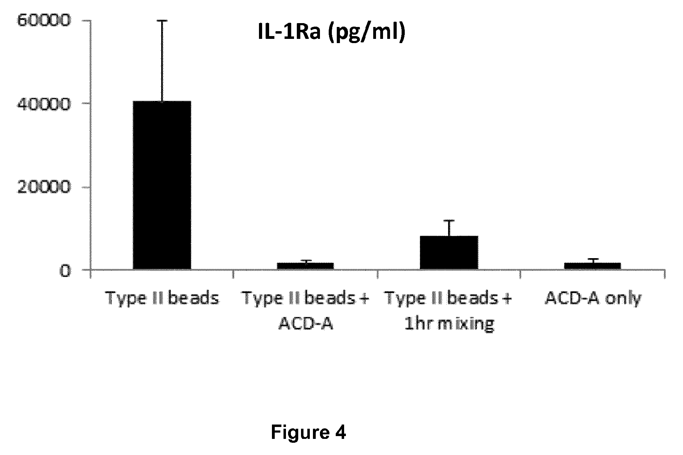

In Figures 4 results shown are IL-1Ra concentration mean values ± SD for the 3 subjects. The y-axis shows the concentration of IL-1Ra in pg/mL, the x axis shows the different groups.

### Results

Below the results from the experimental section are commented.

*Serum obtained via a method according to an embodiment of the invention has a higher concentration of IL-1Ra than serum treated according to a commercial prior-art system.*

As can be seen in Figure 2c serum obtained from a blood sample treated according to an embodiment of the invention (labelled: *Type II beads)* had a mean IL-1Ra concentration of greater than 250 000 pg/mL. The mean concentration of IL-1Ra in serum obtained from a blood sample treated according to a commercial prior art system, Arthrex Vet Systems IRAP II, is shown in Figure 3c and can be seen to be around 45 000 pg/mL (labelled: *IRAP(A)*).

As can be seen in Figure 2d serum obtained from a blood sample treated according to an embodiment of the invention (labelled: *Type II beads*) had furthermore a ratio of IL-1Ra to IL-1β of greater than 3500.

As can be seen in Figure 3d the highest IL-1Ra to IL-1β ratio achieved using non-round bottom (i.e., conical) tubes were only around 900 (labelled: *Type II beads* (B)). Figure 3d also shows that the commercial prior art method resulted in a ratio of IL-1Ra to IL-1β of only around 500.

*Serum obtained via a method according to the invention but wherein the glass beads were treated such that their surface areas were increased had significantly lower concentrations of IL-1Ra compared to those wherein the glass beads had polished surfaces.*

As can be seen in Figure 2c and Figure 2d serum obtained from a blood sample treated in a tube comprising glass beads having being treated to increase their surface area (labelled: *Type I beads; 24 hours*) had a significantly lower concentration of IL-1Ra, and significantly lower ratio of IL-1Ra to IL-1β compared to serum obtained from a blood sample treated according to the process described in experiment 1, where the glass beads had been polished (labelled: *Type II beads*).

This is especially important as it is in contrast to prior art (e.g. in US 6,759,188 B2), which suggests increasing the surface area available to monocytes as an ideal technique to increase anti-inflammatory cytokine production.

*Serum obtained via a method in which the tube had a conical bottom*, *but in which the glass beads had been polished has significantly higher concentration of IL-1Ra than the commercially available prior*-*art system comprising a tube with a conical bottom*.

As can be seen in Figure 3c serum obtained from blood treated in tubes having a conical bottom but comprising polished glass beads (labelled: *Type II beads (B)*) had a significantly higher concentration of IL-1Ra than serum obtained from blood treated to a leading commercially available prior-art system, Arthrex Vet Systems IRAP II (labelled: *IRAP (A)*). The IRAP II system comprises tubes with a conical bottom. In Figure 3c it can furthermore be seen that serum obtained from blood treated in conical bottom tubes comprising glass beads treated such that their surface area was increased (labelled; *Type III beads (C))*had a significantly lower concentration of IL-1Ra than serum obtained from blood treated according to an embodiment of the invention where the tube comprised glass beads having been polished (labelled *Type II beads (B)*).

In confirmation of the statement above, and in contrast to the prevailing prejudice this result indicates that an increase in surface area of any glass beads is not required or even ideal when serum comprising IL-1Ra is to be obtained from blood.

*Serum obtained via a method according to an embodiment of the invention does not have a substantially higher concentration of pro-inflammatory cytokines.*

When comparing Figure 2b with Figure 3b it can be seen that serum obtained from blood treated according to the instructions of a leading commercially available prior art system, Arthrex Vet Systems IRAP II (Figure 3b, labelled: *IRAP(A)*), or the other tubes having a conical bottom (Figure 3b labelled: *Type II beads (B);* and *Type III beads (C))* had a similar, but higher, level of pro-inflammatory cytokine IL-1β compared to serum obtained from round bottom tubes provided with polished beads according to an embodiment of the invention (Figure 2b, labelled: *Type II beads*)*.*

Comparing figures 2a and 3a shows that the concentration of TNFα in serum obtained did not differ significantly regardless of the process or tube used.

*Serum obtained via a method according to an embodiment of the invention has a high ratio of IL-1Ra to IL-1β.*

As can be seen in Figure 2d serum obtained from blood treated according to an embodiment of the invention (labelled: *Type II beads*) has a high ratio of anti-inflammatory cytokines (IL-1Ra) to pro-inflammatory cytokines (IL-1β). This ratio is especially high when compared to the ratios achievable with a leading commercially available prior-art system, Arthrex Vet Systems IRAP II as can be seen in Figure 3d where the IRAP treated sample has a ratio of only around 500 (labelled: *IRAP (A)*).

An improved tube design, wherein the tube has a rounded bottom and is provided with a sealing cap comprising a sealing membrane and a valve is therefore especially relevant for methods for the production of serum for the treatment of bone or tissue diseases or injuries such as orthopaedic diseases.

Furthermore, the results indicate that, in contrast to the prevailing prejudice, any glass beads used in tubes according to the invention are ideally not treated to increase surface area.

*Serum obtained from blood treated with an anti-coagulant has a lower mean concentration of IL-1Ra than blood not treated with an anti-coagulant.*

As can be seen in Figure 4, the results of Experiment 4 show that blood treated with an anti-coagulant had a significantly lower concentration of IL-1Ra than blood not treated with an anti-coagulant. A complete clotting, including platelet retraction, seems to be mandatory in order to achieve serum with anti inflammatory proteins such as IL-IRa.

All-in-all, serum obtained from blood treated according to an embodiment of the invention is an ideal option for the treatment of bone or tissue diseases or injuries such as orthopaedic diseases.

## Claims

1. A method for obtaining serum comprising IL-1Ra from a blood sample, the method comprising the steps of:
- adding a blood sample to a sealed tube configured for receiving a blood sample, wherein the base of the tube is provided with a round-bottom, and wherein the tube comprises a plurality of glass beads having a polished surface and a sealing cap provided with a valve and a sealing membrane;
- agitating the blood sample to induce the production of IL-1Ra; and
- separating the serum comprising IL-1Ra from the blood sample.

2. The method according to claim 1, wherein the blood sample is incubated at 36 to 39 °C once agitated.

3. The method according to claim 2, wherein the incubation is performed for not more than 24 hours.

4. The method according to any one of the claims 1 to 3, wherein the valve is formed by at least one opening in the cap being sealed with a sterile hydrophobic filter having a pore diameter of not more than 0.45µm, such as not more than 0.22µm.

5. The method according to any one of the claims 1 to 4, wherein the beads have an arithmetical mean surface roughness (Ra) of not more than 50nm.

6. The method according to any one of the claims 1 to 5, wherein the agitation is performed by inversion of the tube.

7. The method according to any one of the claims 1 to 6, wherein the plurality of glass beads are treated with an aqueous solution of NaCl before adding the blood sample.

8. The method according to any one of the claims 1 to 7, wherein no further tissue or cellular material is added to the tube apart from the blood sample.

9. The method according to any one of the claims 1 to 8, wherein the tube is pre-heated to a temperature of at least 35 °C prior to the provision of the blood sample.

10. A serum obtainable by the method according to any one of the claims 1 to 9, for use in the treatment of inflammatory or non-inflammatory arthritis in a mammal and/or in the treatment of injuries of tendons and ligaments in a mammal.

11. A serum according to claim 10 having a concentration ratio of IL-1Ra to IL-1β of at least 350.

12. A sealed tube for use in a method according to any one of the claims 1 to 11 for obtaining a serum comprising IL-1Ra from a blood sample, the sealed tube being configured for receiving a blood sample, wherein the base of the tube is provided with a round-bottom, and the tube comprises a sealing cap, the sealing cap being provided with a valve and a sealing membrane.

13. The tube according to claim 12, wherein the tube is further provided with a plurality of glass beads, the glass beads having a polished surface.

14. The tube according to claim 12 or 13, wherein at least one internal surfaces of the tube, and/or the surface of the beads are coated with a tethered-liquid-fluorocarbon (also denoted perfluorocarbon in the art), such as (Heptadecafluoro-1,1,2,2-tetrahydrododecyl)tris(dimetylamino)silane, Trifluoromethyltrimethylsilane, Nonafluorohexyltrimethoxysilane, (Heptadecafluoro-1,1,2,2-tetrahydrodecyl)trimethoxysilane, Trichloro(1H,1H,2H,2H-perfluorooctyl)silane and or perfluorodecalin.

15. The tube according to claim 13 or 14, wherein the glass beads are coated with noble metals such as Au, Ag, Pt, Rh, or Ir, or alloys thereof; with metals forming biocompatible oxides Zr, and Ti, or alloys thereof; with metals forming corrosion resistant oxides Al or Cr or alloys thereof; with W or Mo, or alloys thereof; or with bioactive materials such as Cu, Fe and Co. In some embodiments the glass beads are also coated with oxides such as TiO₂, ZrO₂, Cr₂O₃, Al₂O₃, SnO₂, In₂O₃ and MgO.
